(19) Europäisches Patentamt
European Patent Office
Office européen des brevets

(11) Publication number: **0 394 889 B1**

(12) # EUROPEAN PATENT SPECIFICATION

(45) Date of publication of patent specification: **03.08.94** (51) Int. Cl.$^5$: **C07D 265/02**, A01N 43/72

(21) Application number: **90107606.7**

(22) Date of filing: **21.04.90**

(54) **Heterocyclic diones as pesticides and plant growth regulators.**

(30) Priority: **25.04.89 US 343093**

(43) Date of publication of application:
**31.10.90 Bulletin 90/44**

(45) Publication of the grant of the patent:
**03.08.94 Bulletin 94/31**

(84) Designated Contracting States:
**AT BE CH DE DK ES FR GB GR IT LI LU NL SE**

(56) References cited:
**GB-A- 2 207 425**
**US-A- 4 552 585**

(73) Proprietor: **SANDOZ AG**
**Lichtstrasse 35**
**CH-4002 Basel(CH)**
(84) Designated Contracting States:
**BE CH DK ES FR GB GR IT LI LU NL SE**

(73) Proprietor: **SANDOZ-PATENT-GMBH**
**Humboldtstrasse 3**
**D-79539 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1230 Wien(AT)**
(84) Designated Contracting States:
**AT**

(72) Inventor: **Lee, Shy.Fuh**
**228 Carbonera Avenue**
**Sunnyvale CA 94986(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention concerns substituted 3,5-dioxo-3,4,5,6-tetrahydrooxazinesas herbicides, processes and intermediates for their preparation, compositions containing them and their use as herbicides and acaricides.

USP 4,695,673 describes a wide range of acylated 1,3-dicarbonyl compounds and their use as herbicides but makes no reference to or suggestion of the 3,5-dioxotetrahydrooxazine ring characterizing the compounds of the present invention.

More particularly, the invention concerns compounds of formula Ia

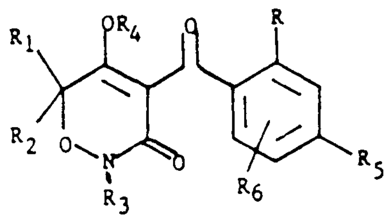

Ia

wherein each of $R_1$, $R_2$ and $R_3$ is independently hydrogen, $C_{1-8}$alkyl, carboxyl, $C_{1-4}$alkoxycarbonyl, phenyl or phenyl substituted by one to three groups selected from the meanings given for R, or $R_1$ and $R_2$ together form a $C_{2-6}$alkylene bridge;

$R_4$ is hydrogen, $C_{1-8}$alkyl, $C_{1-8}$alkylcarbonyl, $C_{1-8}$alkoxycarbonyl, $C(O)NR_7R_8$, $C_{1-8}$alkylsulphonyl, P-(O)($OR_9$)($OR_9'$), $R_7P(O)$-$OR_9$, benzoyl or a cation;

R is $C_{1-8}$alkyl optionally substituted by 1 to 6 halogen atoms, $C_{1-8}$alkoxy optionally substituted by 1 to 6 halogen atoms, $C_{1-8}$alkylcarbonyl, $C_{1-8}$alkoxycarbonyl, $NR_7'R_8'$, $O_nS(O)_{n'}R_{10}$, $NR_7'SO_2R_8'$, halogen, cyano or nitro;

each of $R_5$ and $R_6$ is independently hydrogen or selected from the meanings given for R; or

$R_5$ and $R_6$ together form the group -Y-W-Z- with the proviso that $R_5$ and $R_6$ attach to adjacent carbon atoms of the phenyl ring of the compound of formula Ia;

each of $R_7$, $R_7'$, $R_7''$, $R_8$, $R_8'$ and $R_8''$ is independently hydrogen or $C_{1-8}$alkyl;

each of $R_9$ and $R_9'$ are independently $C_{1-8}$alkyl;

$R_{10}$ is $C_{1-8}$alkyl optionally substituted by 1 to 6 halogen atoms;

each of $R_{11}$, $R_{12}$, $R_{13}$ and $R_{14}$ is independently hydrogen, halogen or $C_{1-8}$alkyl optionally substituted by 1 to 6 halogen atoms;

W is $-(CR_{11}R_{12})_t-(CR_{13}R_{14})_{t'}-$ or sulphonyl;

each of Y and Z is independently oxygen, sulphur, sulphonyl, $CR_7''R_8''$;

n is 0 or 1;

n' is 0, 1 or 2;

t is 1 or 2; and

t' is 0 or 1.

In the above definitions, halogen is conveniently selected from chloro, bromo and fluoro, $C_{1-8}$alkyl moieties, preferably have 1 to 4 carbon atoms.

Each of $R_1$, $R_2$ and $R_3$ is preferably hydrogen, $C_{1-4}$alkyl especially hydrogen or $C_{1-3}$alkyl. Where $R_1$ and $R_2$ together form an alkylene bridge, it is preferably a $C_{3-6}$alkylene bridge.

R conveniently signifies $C_{1-4}$alkyl optionally substituted with halogen, $-(O)_n-S(O)_{n'}-C_{1-4}$alkyl, halogen or nitro. It is preferably methyl, $CF_3$, $C_{1-3}$alkylsulfonyl, $C_{1-3}$alkylsulfonyloxy, chloro, bromo or nitro.

$R_5$ is preferably bromo, chloro, fluoro, trifluoromethyl, $SC_{1-4}$alkyl, $OSO_2C_{1-4}$alkyl, $SO_2C_{1-4}$alkyl, $OSO_2C_{1-4}$haloalkyl, $NR_7'SO_2C_{1-4}$alkyl, or, together with $R_6$, the group -Y-W-Z-. It is more preferably chloro, $C_{1-3}$alkylsulfonyl or $C_{1-3}$alkylsulfonyloxy, or, together with $R_6$, methylenedioxy.

$R_6$ is preferably hydrogen, $C_{1-4}$alkyl, $C_{1-4}$alkoxy, bromo, chloro or, together with $R_5$, the group -Y-W-Z-; it is more preferably hydrogen, methoxy or chloro, or, together with $R_5$, methylenedioxy.

$R_4$ is conveniently hydrogen, $C_{1-4}$alkyl, $C_{4-6}$alkylcarbonyl, benzoyl, $C_{1-4}$alkylsulfonyl or a cation. It is preferably hydrogen, methyl, ethyl, t-butylcarbonyl, isobutylcarbonyl, benzoyl or methylsulfonyl. As a cation $R_4$ is preferably an alkali metal such as $Na^+$, $K^+$, $Li^+$ or an ammonium cation.

$R_1$ and $R_2$ are preferably H, $C_{1-4}$alkyl, phenyl or phenyl substituted by one to three groups selected from the meanings given for R; more preferably H or $C_{1-3}$alkyl e.g. H and methyl;

$R_3$ is preferably $C_{1-8}$alkyl, more preferably $C_{1-4}$alkyl, e.g., $CH_3$ $C_2H_5$;

$R_4$ is preferably H;

R is preferably $NO_2$, Cl, $CF_3$, more preferably $NO_2$ or Cl;

$R_5$ is preferably Cl, Br, F, $CF_3$, $SO_2$-$R_{10}$, $SR_{10}$, $OSO_2R_{10}$, more preferably Cl, $CF_3$, $OSO_2R_{10}$, or $SR_{10}$, e.g., Cl, $CF_3$ or $SCH_3$;

$R_6$ is preferably H;

$R_{10}$ is preferably $C_{1-4}$alkyl optionally halogen substituted, more preferably $C_{1-3}$alkyl optionally halogen substituted, e.g., $C_{1-3}$alkyl.

Preferred groups are those in which $R_1$, $R_2$ and $R_3$ are each methyl and $R_4$ is hydrogen and those in which $R_6$ is hydrogen, R is nitro and $R_5$ is chloro or $SCH_3$. Two particularly preferred compounds are 2,6,6-trimethyl-4-(4-chloro-2-nitrobenzoyl)-2H-1,2-oxazine-3,5-(4H,6H)-dione, and 2,6,6-trimethyl-4-(4-methylthio-2-nitrobenzoyl)-2H-1,2-oxazine-3,5-(4H, 6H)-dione.

The compounds of the present invention of formula Ia are new substances which can be prepared by methods analogous to methods known in the art for the preparation of 2-aroyl-1,3-cycloalkyl-1,3-diones. More particularly, they can be obtained by, for example: rearranging an enol ester of formula IIa

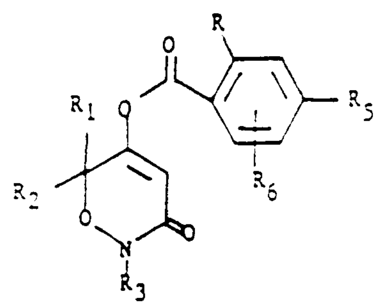

IIa

wherein R-$R_3$, $R_5$ and $R_6$ are as previously defined.

This rearrangement is conveniently effected by reacting the compound of formula IIa with a cyanide source and a moderate base.

For example, the reaction may be carried out in the presence of a catalytic amount of a source of cyanide anion and/or hydrogen cyanide, together with a molar excess, with respect to the enol ester, of a moderate base. The reaction is conveniently carried out in a solvent which is inert under the reaction conditions, e.g. 1,2-dichloroethane, toluene, acetonitrile, methylene chloride, ethyl acetate, dimethylformamide (DMF) and methyl isobutyl ketone (MIBK). In general, depending on the nature of the reactants and the cyanide source, the rearrangement may be conducted at temperatures up to about 80 °C. In some cases, for instance when there is a possible problem of excessive by-product formation, the temperatures should be kept at about 40 °C maximum.

Preferred cyanide sources are alkali metal cyanides such as sodium and potassium cyanide; cyanohydrins of methyl alkyl ketones having from 1-4 carbon atoms in the alkyl groups, such as acetone or methyl isobutyl ketone cyanohydrins; cyanohydrins of benzaldehyde or of $C_2$-$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Among cyanohydrins the preferred cyanide source is acetone cyanohydrin. The cyanide source is used in an amount up to about 50 mole percent based on the enol ester. Generally about 1-10 mol % of the cyanide source is preferred.

By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this reaction include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate. The base is used in an amount of from about 1 to about 4 moles per mole of enol ester, preferably about 1.3-2 moles per mole.

When the cyanide source is an alkali metal cyanide, particularly, potassium cyanide, a phase transfer catalyst may be included in the reaction. Particularly suitable phase transfer catalysts are the Crown ethers.

Compounds of formula I where $R_4$ is as previously defined but excluding H, can be prepared by reacting a compound of formula Ia wherein $R_4$ is H and R-$R_3$, $R_5$ and $R_6$ are as previously defined with either

i) the compound $R_4$-OH and a catalyst, or

ii) the compound $R_4$-Q and a moderate base, wherein Q is a halogen atom.

The above reaction i) is carried out in the presence of a catalyst such as concentrated sulfuric acid. The reaction is conveniently carried out in a solvent which is also the reactant such as methanol, and at an elevated temperature.

The above reaction ii) is carried out in the presence of a moderate base such as triethylamine or pyridine and conveniently at RT or below.

The salt forms of compounds of formula Ia may be prepared by methods known per se, for example by reacting stoichiometric quantities of the compounds of formula Ia wherein $R_4$ is hydrogen with an appropriate base, for example, an alkali metal hydroxide, carbonate or bicarbonate, an alkaline earth metal hydroxide or carbonate, ammonia or an amine (e.g. diethanolamine, triethanolamine, octylamine, morpholine or dioctylamine), in a suitable solvent. Acid addition salts of compounds of general formula Ia which incorporate an amino radical may be prepared from the corresponding compounds of formula Ia by methods known per se, for example by reacting stoichiometric quantities of the compound of formula Ia and the appropriate acid, for example an inorganic acid, e.g. hydrochloric acid, sulphuric acid, phosphoric acid or nitric acid, or an organic acid, e.g. acetic acid, in a suitable solvent. The salts may, if necessary, be purified by recrystallisation from one, two or more suitable solvents.

The compounds of formula Ia may be recovered from the reaction mixture in which they are formed by working up by established procedures.

The compounds of formula IIa may be prepared by reacting a compound of formula III

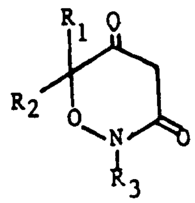

III

with a compound of formula IV

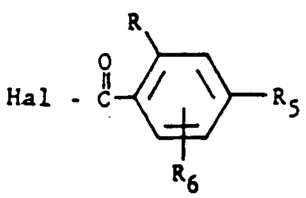

IV

This reaction is carried out in the presence of a base such as triethylamine, potassium carbonate, pyridine, preferably triethylamine and in an inert solvent such as dichloromethane, acetonitrile, toluene, tetrahydrofuran, dimethylformamide. The reaction is conveniently carried out at RT or below.

The compounds of formula III are new and also form part of the invention.

They may be prepared by decarboxylating a compound of formula V

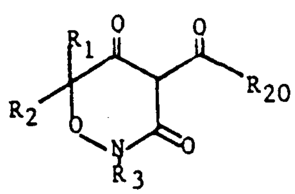

V

wherein $R_{20}$ is alkoxy, especially ethoxy or methoxy and $R_1$, $R_2$ and $R_3$ are as defined above. The reaction may be carried out at elevated temperatures e.g. 80-90° and in an inert solvent such as e.g. wet dimethylsulfoxide.

The compounds of formula V may be prepared analogously to known methods e.g. according to the following reaction scheme.

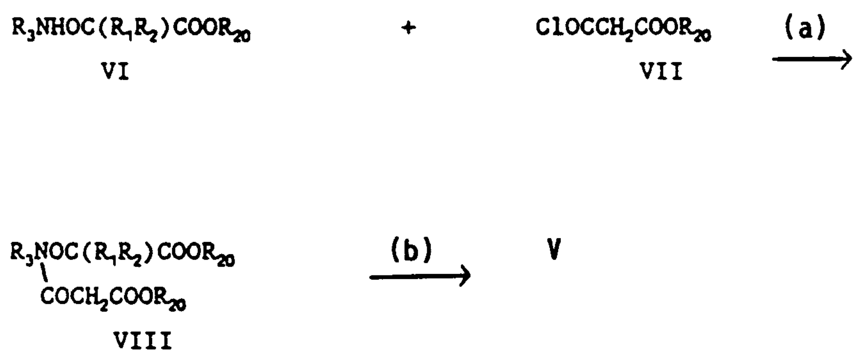


VI VII VIII

wherein $R_{1-3}$ and $R_{20}$ are as previously defined.

Reaction (a) may be carried out in an inert solvent such as dichloromethane and aqueous ether and in the presence of a base such as triethylamine or sodium carbonate at RT.

Reaction (b) may be carried out in an inert solvent such as toluene benzene or tetrahydrofuran in the presence of a base such as sodium methoxide or sodium hydride.

The remaining starting materials and reagents employed in the process described herein are either known or, insofar as they are not known, may be produced in a manner analogous to the processes described herein or to known processes [cf for compounds VI Kornowski et al. Bull. Soc. Chim France 1966(2)683].

The compounds of this invention wherein the OH group is unsubstituted can have four structural formulae because of tautomerism as illustrated as follows for formula Ia where $R_4$ is H:

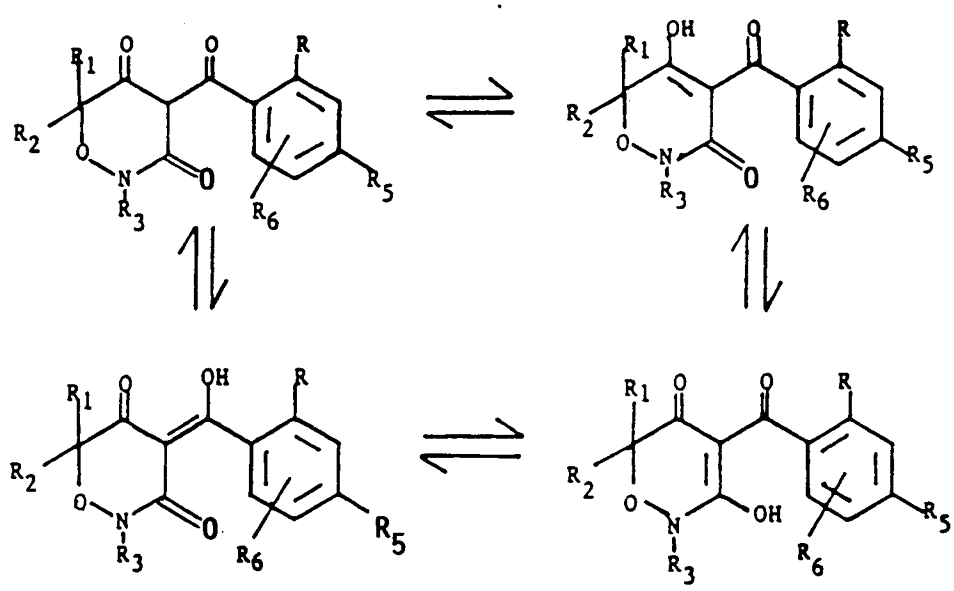

The novel compounds of formula Ia are useful for the control of weeds, using pre- and/or post-emergent treatments. Compounds of formula Ia are also useful as plant growth regulators (PGRs) and acaricides. The compounds can be applied in the form of dusts, granules, solutions, emulsions, wettable powders, flowables and suspensions. Application of a compound of the present invention as herbicides is made according to conventional procedure to the weeds or their locus using an herbicidally effective amount of the compounds, usually from about one-tenth or less to ten pounds per acre (0.11 or less to 11 kg/ha, e.g. 0.05 to 11 kg/ha) more usually 0.05 to 5 kg/ha, and preferably 0.1 to 1 kg/ha, the application being repeated as necessary. The application of a compound of the present invention to the "locus" of the weed includes

application to the seeds, the plant (weed) or parts of the plant, or the soil.

Application of a compound of the present invention as an acaracide is made according to conventional procedure to the site of infestation using an acaricidally effective amount of the compound, usually 100 g/ha to 1 kg/ha.

The term "herbicide", as used herein, refers to an active ingredient which modifies the growth of plants because of phytotoxic or plant growth regulating properties so as to retard the growth of the plant or damage the plant sufficiently to kill it.

The compounds of the present invention, when applied as either post or pre-emergents, demonstrate high levels of herbicidal activity on broadleaf, grass and sedge weeds. They also exhibit selectivity in wheat (e.g. compound 6 in Table A); corn and cotton (e.g. compound 2 in Table A); and rice.

In the use of the compounds of formula Ia for combatting weeds and acari, a compound of formula Ia, or mixture thereof, can conveniently be employed as compositions in association with acceptable diluent(s) for application to the weed, acari or their loci. Such compositions also form part of the present invention.

Methods of preparing suitable formulations which can be used with a compound of the present invention are described in the literature along with suitable liquid or solid carriers. The optimum usage of a compound of the present invention is readily determinable by one of ordinary skill in the art using routine testing such as greenhouse testing and small plot testing.

Suitable formulations contain from 0.01 to 99 % by weight of active ingredient, from 0 to 20 % of surfactant and from 1 to 99.99 % of solid or liquid diluent(s). Higher ratios of surfactant to active ingredient are sometimes desirable and are achieved by incorporation into the formulation or by tank mixing. Application forms of a composition generally contain between 0.01 and 25 % by weight of active ingredient. Lower or higher levels of active ingredient can, of course, be present depending on the intended use, the physical properties of the compound and the mode of application. Concentrate forms of a composition intended to be diluted before use generally contain between 2 and 90 %, preferably between 5 and 80 % by weight of active ingredient.

Useful formulations of the compounds of formula Ia include dusts, granules, suspension concentrates, wettable powders, flowables and the like. They are obtained by conventional manner, e.g. by mixing a compound of formula Ia with the diluent(s) and optionally with other ingredients.

Alternatively, the compounds of formula Ia may be used in micro-encapsulated form.

The compounds of formula Ia can be combined with a cylcodextrin to make a cylcodextrin inclusion complex for application to the weeds, acari or their loci.

Agriculturally acceptable additives may be employed in the herbicidal compositions to improve the performance of the active ingredient and to reduce foaming, caking and corrosion, for example.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

"Diluent" as used herein means a liquid or solid agriculturally acceptable material used to dilute a concentrated material to a usable or desirable strength. For dusts or granules it can be e.g. talc, kaolin or diatomaceous earth, for liquid concentrate forms for example a hydrocarbon such xylene or an alcohol such as isopropanol, and for liquid application forms, e.g. water or diesel oil.

The compositions of this invention can also comprise other compounds having biological activity, e.g. compounds having similar or complementary acaricidal or herbicidal activity for broadspectrum weed control or compounds having antidotal, fungicidal, insecticidal or insect attractant activity.

Typical herbicidal composition, according to this invention, are illustrated by the following Examples A, B and C in which the quantities are in parts by weight.

## EXAMPLE A

### Preparation of a Dust

10 Parts of a compound according to this invention and 90 parts of powdered talc are mixed in a mechanical grinder-blender and are ground until a homogeneous, free-flowing dust of the desired particle size is obtained. This dust is suitable for direct application to the site of the weed infestation.

**EXAMPLE B**

Preparation of Wettable Powder

25 Parts of a compound according to this invention are mixed and milled with 25 parts of synthetic fine silica, 2 parts of sodium lauryl sulphate, 3 parts of sodium ligninsulphonate and 45 parts of finely divided kaolin until the mean particle size is about 5 micron. The resulting wettable powder is diluted with water before use to a spray liquor with the desired concentration.

**EXAMPLE C**

Preparation of Emulsifiable Concentrates (EC)

13.37 Parts of a compound according to this invention are mixed in a beaker with 1.43 parts of Toximul® 360A (a mixture of anionic and non-ionic surfactants containing largely anionic surfactants), 5.61 parts of Toximul® 360B (a mixture of anionic and non-ionic surfactants containing largely non-ionic surfactants), 23.79 parts of dimethyl formamide and 55.8 parts of Tenneco 500-100 (predominantly a mixture of alkylated aromatics such as xylene and ethylbenzene) until solution is effected. The resulting EC is diluted with water for use.

The following Examples are provided to illustrate the practice of the present invention. Temperature is given in degrees Centigrade. RT means room temperature. Parts and percentages are by weight.

FINAL COMPOUNDS

**EXAMPLE I**

Preparation of 2,6-dimethyl-4-(4-chloro-2-nitrobenzoyl)-2H-1,2-oxazine-3,5-(4H,6H)-dione (formula Ia wherein $R_1$ and $R_3$ are $CH_3$; $R_2$, $R_4$ and $R_6$ are H; R is $NO_2$; $R_5$ is Cl; (Compound No. 1 Table A)).

3.65 g of 2,6-dimethyl-5-(4-chloro-2-nitrobenzoyloxy)-6H-1,2-oxazine-3-one is treated at RT with 3.06 ml of triethylamine and 0.3 ml of acetone cyanohydrin in 20 ml of acetonitrile. After stirring overnight the solution is concentrated to a small volume and then taken up in dichloromethane and water. The combined extracts are washed with dilute HCl, brine, dried and evaporated to yield an oily residue. The crude product is recrystallized from ether to give crystalline 2,6-dimethyl-4-(4-chloro-2-nitrobenzoyl)-2H-1,2-oxazine-3,5-(4H,6H)-dione, m.p. 127.5 °C.

Proceeding analogously to Example I the following compounds of formula Ia are obtained.

TABLE A

| Cpd | $R_1$ | $R_2$ | $R_3$ | $R_4$ | R | $R_5$ | $R_6$ | m.p. |
|---|---|---|---|---|---|---|---|---|
| 1 | $CH_3$ | H | $CH_3$ | H | $NO_2$ | 4-Cl | H | 127.5° |
| 2 | $CH_3$ | $CH_3$ | $CH_3$ | H | $NO_2$ | 4-Cl | H | 105° |
| 3 | $CH_3$ | $CH_3$ | $CH_3$ | H | Cl | 4-$SO_2CH_3$ | H | 110° |
| 4 | $CH_3$ | $CH_3$ | $CH_3$ | H | $NO_2$ | 4-Br | H | Foam |
| 5 | $CH_3$ | $CH_3$ | $CH_3$ | H | $NO_2$ | 4-$OSO_2CH_3$ | H | 128° |
| 6 | $CH_3$ | $CH_3$ | $CH_3$ | H | $NO_2$ | 4-$SCH_3$ | H | 104-6° |
| 7 | $CH_3$ | $CH_3$ | $CH_3$ | H | $NO_2$ | 4-F | H | 137° |
| 8 | $CH_3$ | $CH_3$ | $CH_3$ | H | $NO_2$ | 4-$SO_2CH_3$ | H | 124° |
| 9 | $CH_3$ | $CH_3$ | $C_2H_5$ | H | $NO_2$ | 4-Cl | H | Foam |
| 10 | $CH_3$ | $CH_3$ | $C_2H_5$ | H | $NO_2$ | 4-$OSO_2CH_3$ | H | 115° |
| 11 | $CH_3$ | $CH_3$ | $CH_3$ | H | $CF_3$ | 4-F | H | 86° |
| 12 | $CH_3$ | $CH_3$ | $CH_3$ | H | $NO_2$ | 4-$CF_3$ | H | 102-103.5° |
| 13 | $CH_3$ | $CH_3$ | $CH_3$ | H | $NO_2$ | 4-$SO_2CH_2Cl$ | H | |
| 14 | $CH_3$ | $CH_3$ | $CH_3$ | H | $NO_2$ | 4-$SO_2C_2H_5$ | H | |
| 15 | H | H | $C_2H_5$ | H | $NO_2$ | 4-Cl | H | |
| 16 | H | H | n-$C_3H_7$ | H | $NO_2$ | 4-Cl | H | |
| 17 | H | H | n-$C_4H_9$ | H | $NO_2$ | 4-Cl | H | |
| 18 | H | H | n-$C_4H_9$ | H | $NO_2$ | 4-$CF_3$ | H | |
| 19 | $CH_3$ | H | n-$C_3H_7$ | H | $NO_2$ | 4-Cl | H | |
| 20 | $CH_3$ | H | n-$C_3H_7$ | H | $NO_2$ | 4-$CF_3$ | H | |
| 21 | $CH_3$ | $CH_3$ | $CH_3$ | H | $NO_2$ | H | H | 87.5° |
| 22 | $CH_3$ | $CH_3$ | $CH_3$ | H | $NO_2$ | $OCHF_2$ | H | |

NMR Spectra

Compound 4

$^1$H nmr ($CDCL_3$): $\delta$ 1.30, 1.53 (s,s,6H,$C(CH_3)_2$), 3.08, 3.38 (s,s,3H,$NCH_3$) 7.21 (d,1H,8Hz), 7.83 (dd,1H,8Hz), 8.33 (d,1H,2Hz-phenyl H)

Compound 9

$^1$H nmr ($CDCL_3$): $\delta$ 1.33, 1.53 (s,s,6H,$C(CH_3)_2$), 1.20 (m, 3H,$NCH_2\underline{C}H_3$), 3.66 (m,2H,$NC\underline{H}_2CH_3$), 7.28 (d,1H,8Hz), 7.66 (dd,1H,8Hz), 8.18 (d,1H,2Hz-phenyl H).

EXAMPLE 2

Preparation of 2,6-dimethyl-5-(4-chloro-2-nitrobenzoyloxy)-6H-1,2-oxazine-3-one (formula IIa, $R_1 = R_3 = CH_3$, $R_2 = R_6 = H$, $R = NO_2$, $R_5 = Cl$)

To a solution of 1.77 g of 2,6-dimethyl-2H-1,2-oxazine-3,4-(4H,6H)-dione in 15 ml of dichloromethane containing 2.4 ml of triethylamine is added dropwise at 0 °C a solution of 2.72 g of 4-chloro-2-nitrobenzoyl chloride in 10 ml of dichloromethane. After the addition is complete, the reaction mixture is stirred at RT for one hour, then diluted with dichloromethane, washed, dried and evaporated to dryness to give the title compound.

EXAMPLE 3

Preparation of 2,6-dimethyl-2H-1,2-oxazine-3,5-(4H,6H)-dione (Formula III $R_1 = R_3 = CH_3$, $R_2 = H$)

4.9 g of an oily mixture of 2,6-dimethyl-4-methoxycarbonyl-2H-1,2-oxazine-3,5-(4H,6H)-dione and 2,6-dimethyl-4-ethoxycarbonyl-2H-1,2-oxazine-3,5-(4H,6H)-dione is obtained e.g. as illustrated below is heated at 79 °C in 25 ml of DMSO and 0.9 ml of water for 3 hours. The reaction mixture is taken up in ether, poured into water and extracted thoroughly with ether. The combined extracts are dried and evaporated to give 2,6-dimethyl-2H-1,2-oxazine-3,5-(4H,6H)-dione.

The following two diones may be prepared analogously.

2,6,6-trimethyl-2H-1,2-oxazine-3,5-(4H,6H)-dione (Formula III $R_1 = R_2 = R_3 = CH_3$)

2-ethyl-6,6-dimethyl-2H-1,2-oxazine-3,5-(4H,6H)-dione (Formula III $R_1 = R_2 = CH_3$, $R_3 = C_2H_5$).

EXAMPLE 4

Preparation of 2,6-dimethyl-4-methoxycarbonyl-2H-1,2-oxazine-3,5-(4H,6H)-dione and 2,6-dimethyl-4-ethoxycarbonyl-2H-1,2-oxazine-3,5-(4H,6H)-dione as a mixture.

To a suspended solution of sodium methoxide, freshly prepared from 694 mg of sodium metal and methanol, in 45 ml of toluene is added dropwise at RT a solution of 7.1 g of methyl N-ethoxycarbonylacetyl-2-methylaminooxypropionate, in 10 ml of toluene. After completing the addition, the resulting mixture is stirred at RT for 24 hours. The reaction mixture is poured into ice-water and extracted with ether (discarded). The aqueous solution is then acidified with 10% aqueous $HC\ell$ and extracted with dichloromethane. The combined extracts were dried and evaporated to dryness to yield an oily mixture of the title compounds.

EXAMPLE 5

Preparation of methyl N-ethoxycarbonylacetyl-2-methylaminooxypropionate

To a solution of 5.32 g of methyl 2-methylaminooxypropionate in 50 ml of dichloromethane is added dropwise at 0 °C a solution of 6.62 g of ethylmalonyl chloride in 15 ml of dichloromethane. After the addition is complete, the resulting solution is stirred at 0 °C for additional one hour. The reaction mixture is poured into water, and extracted with ether. The combined extracts are washed with dilute $HC\ell$, brine, dried and evaporated to give an oily residue which is chromatographed on silica gel to yield oily methyl N-ethoxycarbonylacetyl-2-methylaminooxypropionate.

NMR spectra for the compounds of examples 2 to 5.

Example 2

$^1H$ nmr ($CDC\ell_3$): $\delta$ 1.47 (d,3H,OCH($C\underline{H}_3$)), 3.20 (s,3H,$NCH_3$), 4.81 (q,1H,OC$\underline{H}$($CH_3$)), 6.15 (s,1H, =CHCO) and 7.77, 7.97 (s,dd,3H,phenyl H).

Example 3

$^1H$ nmr ($CDC\ell_3$): $\delta$ 1.43 (d,3H,OCH($C\underline{H}_3$)), 3.30 (S,1H,$NCH_3$), 3.53 (q,2H,$OCCH_2CO$) and 4.40 (q,1H,OC$\underline{H}_3$)).

Example 4

2,6-dimethyl-4-carbomethoxy-2H-1,2-oxazine-3,5(4H,6H)-dione

$^1$H nmr ($CDCL_3$): δ 1.50 (d,3H,OCH($CH_3$)), 3.21 (s,3H,$NCH_3$), 3.93 (2,3H,$OCH_3$), and 4.73 (q,1H,OCH-($CH_3$)).

2,6-dimethyl-4-carboethoxy-2H-1,2-oxazine-3,5(4H,6H)-dione

$^1$H nmr ($CDC\ell_3$): δ 1.40 (t,3H,$OCH_2CH_3$), 1.48 (d,3H,OCH($CH_3$)), 3.21 (s,3H,$NCH_3$), 4.41 (q,2H,$OCH_3CH_3$) and 4.73 (q,1H,OCH($CH_3$)).

Example 5

Methyl N-ethoxycarbonylacetyl-2-methylaminooxypropionate

$^1$H nmr ($CDC\ell_3$): δ 1.28 (t,3H,$OCH_2CH_3$), 3.23 (s,3H,$NCH_3$), 3.70 (q,2H,$OCCH_2CO$), 3.77 (s,3H,$OCH_3$), 4.21 (q,2H,$OCH_2CH_3$) and 4.57 (q,1H,OCH($CH_3$).

**Claims**

**1.** A compound having the formula Ia

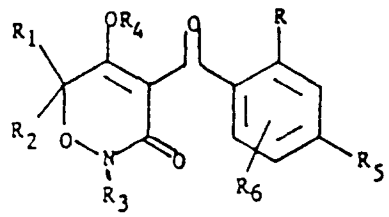

Ia

wherein each of $R_1$, $R_2$ and $R_3$ is independently hydrogen, $C_{1-8}$alkyl, carboxyl, $C_{1-4}$alkoxycarbonyl, phenyl or phenyl substituted by one to three groups selected from the meanings given for R, or $R_1$ and $R_2$ together form a $C_{2-6}$alkylene bridge;

$R_4$ is hydrogen, $C_{1-8}$alkyl, $C_{1-8}$alkylcarbonyl, $C_{1-8}$alkoxycarbonyl, $C(O)NR_7R_8$, $C_{1-8}$alkylsulphonyl, $P(O)$-$(OR_9)(OR_9')$, $R_7P(O)$-$OR_9$, benzoyl or a cation;

R is $C_{1-8}$alkyl optionally substituted by 1 to 6 halogen atoms, $C_{1-8}$alkoxy optionally substituted by 1 to 6 halogen atoms, $C_{1-8}$alkylcarbonyl, $C_{1-8}$alkoxycarbonyl, $NR_7'R_8'$, $O_nS(O)_{n'}R_{10}$, $NR_7'SO_2R_8'$, halogen, cyano or nitro;

each of $R_5$ and $R_6$ is independently hydrogen or selected from the meanings given for R; or

$R_5$ and $R_6$ together form the group -Y-W-Z- with the proviso that $R_5$ and $R_6$ attach to adjacent carbon atoms of the phenyl ring of the compound of formula Ia;

each of $R_7$, $R_7'$, $R_7''$, $R_8$, $R_8'$ and $R_8''$ is independently hydrogen or $C_{1-8}$alkyl;

each of $R_9$ and $R_9'$ are independently $C_{1-8}$alkyl;

$R_{10}$ is $C_{1-8}$alkyl optionally substituted by 1 to 6 halogen atoms;

each of $R_{11}$, $R_{12}$, $R_{13}$ and $R_{14}$ is independently hydrogen, halogen or $C_{1-8}$alkyl optionally substituted by 1 to 6 halogen atoms;

W is $-(CR_{11}R_{12})_t-(CR_{13}R_{14})_{t'}-$ or sulphonyl;

each of Y and Z is independently oxygen, sulphur, sulphonyl or $CR_7''R_8''$;

n is 0 or 1;

n' is 0, 1 or 2;

t is 1 or 2; and

t' is 0 or 1.

**2.** A compound according to Claim 1, wherein

each of $R_1$, $R_2$, $R_3$ is independently selected from hydrogen or $C_{1-4}$alkyl;

R is selected from $C_{1-4}$alkyl optionally substituted with halogen, $-(O)_n-S(O)_{n'}-C_{1-4}$alkyl, halogen or nitro;

$R_5$ is selected from bromo, chloro, fluoro, trifluoromethyl, $SC_{1-4}$alkyl, $OSO_2C_{1-4}$alkyl, $SO_2C_{1-4}$al-

kyl, $OSO_2$-$C_{1-4}$haloalkyl, $NR_7$'$SO_2C_{1-4}$alkyl, or, together with $R_6$ the group -Y-W-Z-;

$R_6$ is selected from hydrogen, $C_{1-4}$alkyl, $C_{1-4}$alkoxy, bromo, chloro or, together with $R_5$, the group -Y-W-Z-;

and $R_4$ is selected from H, $C_{1-4}$alkyl, $C_{4-8}$alkylcarbonyl, benzoyl, $C_{1-4}$alkylsulphonyl or a cation.

**3.** A compound according to Claim 1 wherein

each of $R_1$ and $R_2$ is independently selected from H, $C_{1-4}$alkyl, phenyl or phenyl substituted by one to three groups selected from the meanings given for R;

$R_3$ is $C_{1-8}$alkyl;

$R_4$ is H;

R is selected from $NO_2$ and Cl;

$R_5$ is selected from Cl, Br, F, $CF_3$, $SO_2R_{10}$, $SR_{10}$ and $OSO_2R_{10}$;

$R_6$ is H;

and $R_{10}$ is selected from $C_{1-4}$alkyl optionally halogen substituted.

**4.** A compound according to Claim 3, wherein $R_1$, $R_2$ and $R_3$ are each methyl, $R_4$ and $R_6$ are each hydrogen, R is nitro and $R_5$ is chloro or $-SCH_3$.

**5.** A pesticidal composition comprising the compound of formula Ia as defined in Claims 1 to 4, in association with an agriculturally acceptable carrier.

**6.** A method of controlling weeds comprising applying to the weeds or their locus a herbicidally effective amount of a compound of formula Ia as defined in Claims 1 to 4.

**7.** A process for preparing a compound of the formula Ia

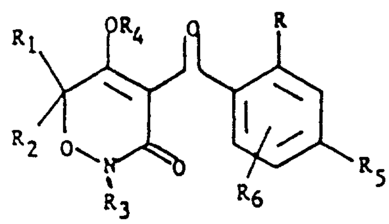

Ia

wherein R-$R_3$, $R_5$ and $R_6$ are as defined in Claim 1, which comprises

a) when $R_4$ is H, rearranging an enol ester of formula IIa

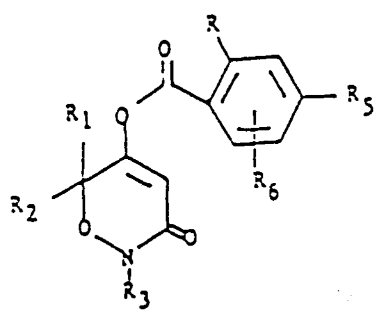

IIa

wherein R-$R_3$, $R_5$ and $R_6$ are as defined above; or

b) when $R_4$ is as defined in Claim 1 but excluding H, reacting a compound of formula Ia wherein $R_4$ is H and R-$R_3$, $R_5$ and $R_6$ are as defined in Claim 1 with either

i) the compound $R_4$-OH and a catalyst, or

ii) the compound $R_4$-Q and a moderate base, wherein Q is a halogen atom and $R_4$ is as defined in Claim 1 but excluding H.

**8.** A compound of formula III

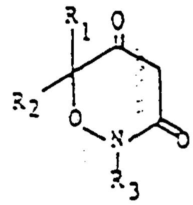

III

wherein $R_1$, $R_2$ and $R_3$ are as defined in Claim 1.

**Patentansprüche**

**1.** Eine Verbindung der Formel Ia

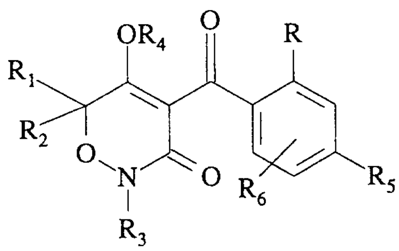

Ia

worin $R_1$, $R_2$ und $R_3$ jeweils unabhängig voneinander Wasserstoff, $C_{1-8}$Alkyl, Carboxyl, $C_{1-4}$Alkoxycarbonyl, Phenyl oder durch ein bis drei Gruppen ausgewählt unter denen für R angegebenen Bedeutungen substituiertes Phenyl, oder
$R_1$ und $R_2$ zusammen eine $C_{2-6}$Alkylen-Brücke,
$R_4$ Wasserstoff, $C_{1-8}$Alkyl, $C_{1-8}$Alkylcarbonyl, $C_{1-8}$Alkoxycarbonyl, $C(O)NR_7R_8$, $C_{1-8}$ Alkylsulfonyl, $P(O)(OR_9)(OR_9')$, $R_7P(O)$-$OR_9$, Benzoyl oder ein Kation,
R gegebenenfalls durch 1 bis 6 Halogenatome substituiertes $C_{1-8}$Alkyl, gegebenenfalls durch 1 bis 6 Halogenatome substituiertes $C_{1-8}$Alkoxy, $C_{1-8}$Alkylcarbonyl, $C_{1-8}$Alkoxycarbonyl, $NR_7'R_8'$, $O_nS(O)_{n'}R_{10}$, $NR_7'SO_2R_8'$, Halogen, Cyan oder Nitro,
$R_5$ und $R_6$ unabhängig voneinander Wasserstoff oder eine der für R gegebenen Bedeutungen, oder
$R_5$ und $R_6$ gemeinsam die Gruppe -Y-W-Z- mit der Massgabe, dass $R_5$ und $R_6$ jeweils an benachbarte Kohlenstoffatome des Phenylrings der Verbindung der Formel Ia gebunden sind,
$R_7$, $R_7'$, $R_7''$, $R_8$, $R_8'$, $R_8''$ jeweils unabhängig voneinander Wasserstoff oder $C_{1-8}$Alkyl,
$R_9$ und $R_9'$ jeweils unabhängig voneinander $C_{1-8}$Alkyl,
$R_{10}$ gegebenenfalls durch 1 bis 6 Halogenatome substituiertes $C_{1-8}$Alkyl,
$R_{11}$, $R_{12}$, $R_{13}$ und $R_{14}$ jeweils unabhängig voneinander Wasserstoff, Halogen oder gegebenenfalls durch 1 bis 6 Halogenatome substituiertes $C_{1-8}$Alkyl,
W die Gruppe $-(CR_{11}R_{12})_t-(CR_{13}R_{14})_{t'}-$ oder Sulfonyl,
Y und Z jeweils unabhängig voneinander Wasserstoff, Schwefel, Sulfonyl oder $CR_7''R_8''$,
n null oder 1,
n' null, 1 oder 2,
t 1 oder 2, und
t' 0 oder 1 bedeuten.

**2.** Verbindung gemäss Anspruch I, worin
$R_1$, $R_2$, $R_3$ unabhängig voneinander Wasserstoff oder $C_{1-4}$Alkyl;
R gegebenenfalls durch Halogen substituiertes $C_{1-4}$Alkyl, $-(O)_n-S(O)_{n'}-C_{1-4}$Alkyl, Halogen oder Nitro.
$R_5$ Brom, Chlor, Fluor, Trifluormethyl, $SC_{1-4}$Alkyl, $OSO_2C_{1-4}$Alkyl, $SO_2C_{1-4}$Alkyl, $OSO_2$-$C_{1-4}$Halogenalkyl, $NR_7'SO_2C_{1-4}$Alkyl oder gemeinsam mit $R_6$ die Gruppe -Y-W-Z-;
$R_6$ Wasserstoff, $C_{1-4}$Alkyl, $C_{1-4}$Alkoxy, Brom, Chlor oder gemeinsam mit $R_5$ die Gruppe -Y-W-Z-; und
$R_4$ Wasserstoff, $C_{1-4}$Alkyl, $C_{4-8}$Alkylcarbonyl, Benzoyl, $C_{1-4}$Alkylsulfonyl oder ein Kation bedeuten.

**3.** Verbindung gemäss Anspruch 1, worin
$R_1$ und $R_2$ unabhängig voneinander Wasserstoff, $C_{1-4}$Alkyl, Phenyl oder durch 1 bis 3 Substituenten ausgewählt unter den Bedeutungen von R substituiertes Phenyl;
$R_3$ $C_{1-8}$Alkyl;
$R_4$ Wasserstoff;
R $NO_2$ oder Cl;
$R_5$ Cl, Br, F, $CF_3$, $SO_2R_{10}$, $SR_{10}$, oder $OSO_2R_{10}$;
$R_6$ Wasserstoff; und
$R_{10}$ gegebenenfalls durch Halogen substituiertes $C_{1-4}$Alkyl bedeuten.

**4.** Verbindungen gemäss Anspruch 3, worin $R_1$, $R_2$ und $R_3$ jeweils Methyl, $R_4$ und $R_6$ jeweils Wasserstoff, R Nitro und $R_5$ Chlor oder $SCH_3$ bedeuten.

**5.** Pestizides Mittel, enthaltend eine Verbindung der Formel Ia gemäss Ansprüchen 1 bis 4 zusammen mit einem in der Landwirtschaft annehmbaren Trägermaterial.

**6.** Verfahren zur Kontrolle von Unkräutern, das darin besteht, dass man die Unkräuter oder ihren Standort mit einer herbizid wirksamen Menge einer Verbindung der Formel Ia gemäss Ansprüchen 1 bis 4 behandelt.

**7.** Verfahren zur Herstellung einer Verbindung der Formel Ia

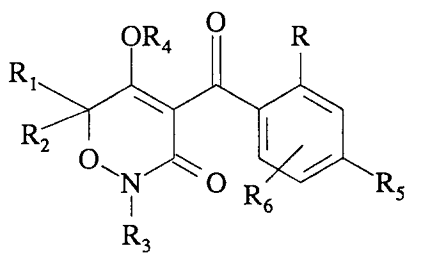

Ia

worin R-$R_3$, $R_5$ und $R_6$ wie im Anspruch 1 definiert sind, dadurch gekennzeichnet, dass man
a) wenn $R_4$ für Wasserstoff steht, einen Enolester der Formel IIa

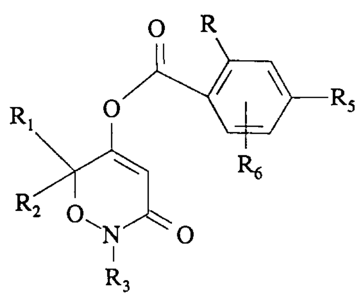

IIa

worin R-$R_3$, $R_5$ und $R_6$ wie oben definiert sind, umlagert; oder
b) wenn $R_4$ mit Ausnahme von Wasserstoff wie im Anspruch 1 definiert ist, eine Verbindung der Formel Ia, worin $R_4$ Wasserstoff bedeutet und R-$R_3$, $R_5$ und $R_6$ wie im Anspruch 1 definiert sind, entweder
i) mit einer Verbindung $R_4$-OH und einem Katalysator, oder
ii) mit einer Verbindung $R_4$-Q und einer milden Base, worin Q ein Halogenatom ist und $R_4$ mit Ausnahme von Wasserstoff wie im Anspruch 1 definiert ist, umsetzt.

8. Eine Verbindung der Formel III

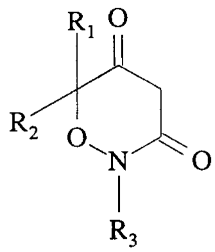

III

worin $R_1$, $R_2$ und $R_3$ wie im Anspruch 1 definiert sind.

## Revendications

1. Un composé répondant à la formule Ia

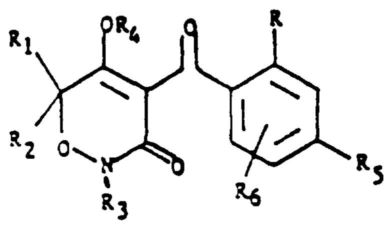

Ia

dans laquelle chaque $R_1$, $R_2$ et $R_3$ signifie indépendamment l'hydrogène ou un groupe alkyle en $C_1$-$C_8$, carboxy, alcoxy en $C_1$-$C_4$ -carbonyle, phényle ou phényle substitué par 1 à 3 groupes choisis parmi les significations données pour R, ou bien $R_1$ et $R_2$ forment ensemble un pont alkylène en $C_2$-$C_6$,
$R_4$ signifie l'hydrogène ou un groupe alkyle en $C_1$-$C_8$, alkyl en $C_1$-$C_8$ -carbonyle, alcoxy en $C_1$-$C_8$ -carbonyle, $C(O)NR_7R_8$, alkylsulfonyle en $C_1$-$C_8$,
$P(O)$-$(OR_9)(OR_9')$, $R_7P(O)$-$OR_9$, benzoyle ou un cation,
R signifie un groupe alkyle en $C_1$-$C_8$ éventuellement substitué par 1 à 6 atomes d'halogène, un groupe alcoxy en $C_1$-$C_8$ éventuellement substitué par 1 à 6 atomes d'halogène, un groupe alkyl en $C_1$-$C_8$-carbonyle, alcoxy en $C_1$-$C_8$ -carbonyle, $NR_7'R_8'$, $O_nS(O)_{n'}R_{10}$, $NR_7'SO_2R_8'$, un halogène ou un groupe cyano ou nitro, chaque $R_5$ et $R_6$ signifie indépendamment l'hydrogène ou est choisi parmi les significations données pour R, ou bien
$R_5$ et $R_6$ forment ensemble le groupe -Y-W-Z-, $R_5$ et $R_6$ devant être fixés à des atomes de carbone adjacents du cycle phényle du composé de formule Ia, chaque $R_7$, $R_7'$, $R_7''$, $R_8$, $R_8'$ et $R_8''$ signifie indépendamment l'hydrogène ou un groupe alkyle en $C_1$-$C_8$,
chaque $R_9$ et $R_9'$ signifie indépendamment un groupe alkyle en $C_1$-$C_8$,
$R_{10}$ signifie un groupe alkyle en $C_1$-$C_8$ éventuellement substitué par 1 à 6 atomes d'halogène,
chaque $R_{11}$, $R_{12}$, $R_{13}$ et $R_{14}$ signifie indépendamment l'hydrogène, un halogène ou un groupe alkyle en $C_1$-$C_8$ éventuellement substitué par 1 à 6 atomes d'halogène,
W signifie $-(CR_{11}R_{12})_t-(CR_{13}R_{14})_{t'}-$ ou un groupe sulfonyle,
chaque Y et Z signifie indépendamment l'oxygène, le soufre, un groupe sulfonyle ou $CR_7''R_8''$,
n signifie 0 ou 1,
n' signifie 0, 1 ou 2,
t signifie 1 ou 2, et
t' signifie 0 ou 1.

2. Un composé selon la revendication 1, dans lequel
chaque $R_1$, $R_2$, $R_3$ est choisi indépendamment parmi l'hydrogène et les groupes alkyle en $C_1$-$C_4$,
R est choisi parmi les groupes alkyle en $C_1$-$C_4$ éventuellement substitués par de l'halogène, $-(O)_n$-$S(O)_{n'}$-alkyle en $C_1$-$C_4$, les halogènes et le groupe nitro,
$R_5$ est choisi parmi le brome, le chlore, le fluor et les groupes trifluorométhyle, S-alkyle en $C_1$-$C_4$, $OSO_2$-alkyle en $C_1$-$C_4$, $SO_2$-alkyle en $C_1$-$C_4$, $OSO_2$-haloalkyle en $C_1$-$C_4$ et $NR_7'SO_2$-alkyle en $C_1$-$C_4$,

ou forme, ensemble avec $R_6$, le groupe -Y-W-Z-,
$R_6$ est choisi parmi l'hydrogène, les groupes alkyle en $C_1$-$C_4$, alcoxy en $C_1$-$C_4$, le brome et le chlore, ou forme, ensemble avec $R_5$, le groupe -Y-W-Z-, et
$R_4$ est choisi parmi H et les groupes alkyle en $C_1$-$C_4$, alkyl en $C_4$-$C_8$-carbonyle, benzoyle et alkyl en $C_1$-$C_4$-sulfonyle ou signifieun cation.

**3.** Un composé selon la revendication 1, dans lequel chaque $R_1$ et $R_2$ est choisi indépendamment parmi H et les groupes alkyle en
$C_1$-$C_4$, phényle ou phényle substitué par 1 à 3 groupes choisis parmi les significations données pour R,
$R_3$ signifie un groupe alkyle en $C_1$-$C_8$,
$R_4$ signifie H,
R est choisi parmi $NO_2$ et Cl,
$R_5$ est choisi parmi Cl, Br, F, $CF_3$, $SO_2R_{10}$ , $SR_{10}$ et $OSO_2R_{10}$,
$R_6$ signifie H, et
$R_{10}$ est choisi parmi les groupes alkyle en $C_1$-$C_4$ éventuellement substitués par de l'halogène.

**4.** Un composé selon la revendication 3, dans lequel $R_1$, $R_2$ et $R_3$ signifient chacun un groupe méthyle, $R_4$ et $R_6$ signifient chacun l'hydrogène, R signifie un groupe nitro et $R_5$ signifie le chlore ou $-SCH_3$.

**5.** Une composition pesticide comprenant le composé de formule Ia tel que défini aux revendications 1 à 4, en association avec un véhicule acceptable en agriculture.

**6.** Une méthode pour combattre les mauvaises herbes, qui comprend l'application sur les mauvaises herbes ou leur zone de croissance d'une quantité efficace du point de vue herbicide d'un composé de formule Ia tel que défini aux revendications 1 à 4.

**7.** Un procédé de préparation d'un composé de formule Ia

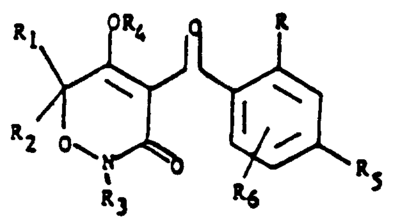

Ia

dans laquelle R-$R_3$, $R_5$ et $R_6$ sont tels que définis à la revendication 1, qui comprend
a) lorsque $R_4$ signifie H, le réarrangement d'un ester énolique de formule IIa

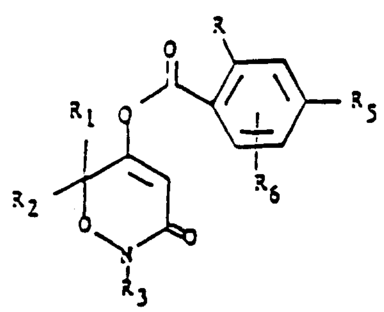

IIa

dans laquelle R-$R_3$, $R_5$ et $R_6$ sont tels que définis plus haut, ou
b) lorsque $R_4$ est tel que défini à la revendication 1 mais à l'exclusion de H, la réaction d'un composé de formule Ia dans laquelle $R_4$ signifie H et R-$R_3$, $R_5$ et $R_6$ sont tels que définis à la revendication 1 avec
i) le composé $R_4$-OH et un catalyseur, ou

ii) le composé $R_4$-Q et une base modérée, où Q signifie un atome d'halogène et $R_4$ est tel que défini à la revendication 1 mais à l'exculsion de H.

**8.** Un composé de formule III

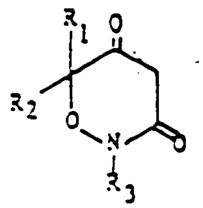

III

dans laquelle $R_1$, $R_2$ et $R_3$ sont tels que définis à la revendication 1.